# EUROPÄISCHE PATENTSCHRIFT

(11) **EP 2 440 265 B1**
(45) Veröffentlichungstag und Bekanntmachung des Hinweises auf die Patenterteilung: **17.12.2014**
(21) Anmeldenummer: 10721825.7
(22) Anmeldetag: 09.06.2010
(51) Int. Cl.: A61M 1/16

(54) **DIALYSEVORRICHTUNG**
DIALYSIS DEVICE
DISPOSITIF DE DIALYSE

(30) Priorität: 10.06.2009 DE 102009026901
(43) Veröffentlichungstag der Anmeldung: 18.04.2012
(73) Patentinhaber: Fresenius Medical Care Deutschland GmbH, 61352 Bad Homburg (DE)
(72) Erfinder: HERRENBAUER, Michael, 61267 Neu-Anspach (DE); POHLMEIER, Robert, 61350 Bad Homburg (DE)
(74) Vertreter: Molnia, David
(86) Internationale Anmeldenummer: PCT/EP2010/058071
(87) Internationale Veröffentlichungsnummer: WO 2010/142717

(56) Entgegenhaltungen:
- WO-A2-2004/082733
- WO-A2-2006/088419
- DE-A1- 19 540 079
- US-A- 3 799 873
- US-A- 5 660 722
- US-A1- 2006 041 216

## Beschreibung

Die Erfindung betrifft eine Dialysevorrichtung gemäß dem Oberbegriff des Anspruchs 1 .

Dialysevorrichtungen der hier angesprochenen Art sind bekannt und werden zur Blutreinigung angewendet, wenn es zu einem Nierenversagen kommt. Insbesondere bei akutem Nierenversagen ist die Entfernung von mittelgroßen Molekülen, wie z.B. Myoglobin oder Interleukine, auch Mittelmoleküle genannt, besonders wichtig. Derartige Mittelmoleküle weisen ein Molekulargewicht von bspw. 1.000 bis 50.000 Da (Dalton) auf. Hingegen ist in diesem Fall die Entfernung von kleineren Molekülen, d.h. Moleküle mit einem geringeren Molekulargewicht unter 1000 Da auf einem definierten Niveau ausreichend. Entscheidend bei akutem Nierenversagen ist also auch die effiziente Entfernung von Mittelmolekülen.

Bei den Dialyseverfahren wird im Wesentlichen zwischen der Hämofiltration und der Hämodialyse unterschieden. Während bei der Hämodialyse ein Stoffaustausch und damit die Reinigung des Bluts hauptsächlich durch Diffusion erfolgen, wird bei der Hämofiltration die Reinigung durch einen konvektiven Stofftransport erreicht. Es hat sich gezeigt, dass bei einem konvektiven Stofftransport die Clearance für Mittelmoleküle größer ist, als bei einem diffusiven Stofftransport, wobei die Clearance das Volumen angibt, welches pro Zeiteinheit von der entsprechenden Substanz entfernt wird. Üblicherweise wird bei akutem Nierenversagen ein Hämofiltrationsverfahren gegenüber einer Hämodialyse bevorzugt. Das Hämofiltrationsverfahren ist jedoch gegenüber der Hämodialyse mit einigen gravierenden Nachteilen verbunden.

So wird bei der Hämofiltration das abgefilterte Blutplasma durch eine Substitutionslösung ersetzt, wobei ca. 1 - 8 Liter Substitutionslösung pro Stunde verbraucht werden. Diese Lösung muss jedoch üblicherweise in Form von 4,5 - 5 Liter Beuteln vom Personal zu dem Behandlungsplatz transportiert werden. Die Beutel müssen anschließend auch entsorgt werden, wodurch sich ein enormer Arbeitsaufwand und hohe Kosten ergeben.

Weiterhin sind für die Hämofiltration ausreichend hohe Blutflüsse notwendig. Patienten, die unter einem chronischen Nierenversagen leiden, verfügen in der Regel über einen so genannten arteriovenösen Shunt, d.h. einen künstlichen Zugang, der Blutflüsse zwischen 300 - 400 ml/min ermöglicht. Bei Patienten mit akutem Nierenversagen steht ein solcher Zugang selten zur Verfügung, weshalb oft nur Flüsse von 100 - 200 ml/min erreicht werden. Der für die effiziente Hämofiltration notwendige hohe Blutfluss verursacht typischerweise bei diesen Patienten typischerweise während der Behandlung zahlreiche Alarme wegen Gefäßzugangsproblemen. Diese Alarme müssen dann vom Klinikpersonal behoben werden, was zu einem zusätzlichen Arbeitsaufwand führt.

Ein weiterer Nachteil der Hämofiltration ist der konvektive Transport, der im Vergleich zum diffusiven Austausch bei der Hämodialyse ein schnelleres Verstopfen des Dialysefilters, das sogenannte Filter-Clogging, verursacht. In der Langzeit-Behandlung müssen die Filter somit öfter ausgetauscht werden, was wiederum Kosten verursacht und einen größeren Arbeitsaufwand für die Behandlung bedeutet.

Schließlich liegt ein weiterer Nachteil der Hämofiltration darin, dass der Patient während der Behandlung je nach verwendetem Hämofilter einen wesentlich höheren Albumin-Verlust erleiden kann als bei der Hämodialyse. Ferner unterliegen die bei der Hämofiltration benötigten Substitutionslösungen dem Arzneimittelgesetz, was die Flexibilität bzgl. Individualisiesrung in der Praxis begrenzt.

Insgesamt zeigt sich, dass die Arbeitsbelastung des Personals und die Kosten der Behandlung bei der Hämofiltration sehr hoch sind, sodass dieses Behandlungsverfahren im Regelbetrieb praktisch relevante Limitierungen hat.

Aus dem Stand der Technik sind bereits Vorrichtungen und Verfahren vorgeschlagen worden, um dennoch eine hohe Clearance von mittelgroßen Molekülen bei geringem Lösungseinsatz und damit geringen Kosten und reduzierten Arbeitsaufwand zu erzielen. Beispielsweise ist aus der WO 2004/082733 ein Hämofiltrationsverfahren bekannt, bei dem zwei Filter kaskadenförmig angeordnet sind. Dabei wird das im ersten Filter gewonnene Filtrat in dem zweiten Filter erneut filtriert. Das in dem zweiten Filter gewonnene Filtrat wird dann als Substitutionslösung dem Blut des Patienten in der Prädilution wieder zugeführt. Dadurch, dass der zweite Filter Mittelmoleküle in dem ersten Filtrat zurück hält, wird dem Blut eine Substitutionslösung zugeführt, die eine verminderte Anzahl an Mittelmolekülen aufweist. Dadurch wird zwar die Menge an benötigter Substitutionslösung vermindert und die Anzahl an mittelgroßen Molekülen im Blut reduziert, nachteilig ist jedoch, dass durch die Verdünnung des Blutes vor der Filtration die Behandlungseffizienz bzgl. der Mittelmoleküle nicht optimal ist.

Aus der WO 2006/088419 A2 ist ein System und ein Verfahren für die Regenerierung einer Dialyseflüssigkeit bekannt.

Aufgabe der Erfindung ist es daher, eine behandlungseffiziente Dialysevorrichtung zu schaffen, die kostengünstig ist und die eine effiziente Entfernung von mittelgroßen Molekülen auch bei einem geringeren Blutfluss mit geringem Arbeitsaufwand bewirkt.

Zur Lösung dieser Aufgabe wird eine Dialysevorrichtung mit den Merkmalen des Anspruchs 1 vorgeschlagen. Die Dialysevorrichtung weist dabei einen Dialysatkreislauf auf, welcher einen ersten Dialysator und einen zweiten Dialysator beinhaltet. Dabei ist der erste Dialysator über eine Blutzuführung und eine Blutrückführung mit dem Blutkreislauf eines Patienten verbindbar. Der zweite Dialysator weist eine Filtermembran mit einem Cut-Off-Wert von mindestens 500 Da auf und der erste Dialysator hat eine Filtermembran mit einem höheren Cut-Off-Wert als der zweite Dialysator.

Das Retentat ist der Anteil des Dialysats, welcher in den zweiten Dialysator geleitet wird und dort die Membran nicht durchdringt, sondern aus dem Dialysatkreislauf entfernt und entsorgt wird. Unter einer Filtermembran wird die Membran eines beliebigen Flüssigkeitsfilter verstanden, welcher geeignet ist, einen Anteil des Stroms abhängig von der Teilchen- (oder Molekül-)-Größe herauszufiltern. Da am zweiten Dialysator vorzugsweise die zugeführte Menge der Filtrationszufuhr - wie später im Detail erläutert - in zwei Ströme aufgeteilt wird, kann der zweite Dialysator auch als ein Dialysierfilter bezeichnet werden.

Ein Cut-Off ist ein Parameter der Filtermembran, welcher sich auf ihre Porengröße bezieht. Moleküle, deren Masse größer ist, als ein entsprechender Cut-Off des Filters, können die Membran des Filters nicht oder nur in einem zu vernachlässigend geringen Verhältnis durchdringen. In diesem Sinne kann der genannte Cut-Off von 500 Da als eine maximale Porengröße des Filters von 0,001 - 0,005 µm verstanden werden. Beispielsweise weisen Elektrolyte (wie Ca-, Na-, Phosphat-, oder ähnliche Ionen) eine Masse in der Größenordnung von 100 Da auf. Da der Cut-Off des zweiten Dialysators deutlich größer als diese Elektrolytgröße ist, werden diese im zweiten Dialysator nicht zurückgehalten und sorgen dafür, wie später im Detail erläutert wird, dass vor allem Moleküle einer Mittelgröße dem Blutstrom entfernt werden. Alternativ zu der im Anspruch gewählten Formulierung des Cut-Off-Werts von 500 Da wird die Erfindung auch so verstanden, dass im Permetatstrom des zweiten Dialysator die Elektrolytkonzentration in Bezug auf seine Filtrationszufuhr, welche die Menge ist, die dem zweiten Dialysator zugeführt wird, im Wesentlichen unverändert bleibt.

Hieraus ergibt sich, wie später noch im Detail erläutert wird, dass verstärkt Moleküle einer Mittelgröße von 1 - 50 kDa und insbesondere bevorzugt 5 - 25 kDa dem Blutstrom entzogen werden.

Insgesamt werden die vorgenannten Vorteile der Hämofiltration auch mit der vorgeschlagenen Dialysevorrichtung weitgehend realisiert und gleichzeitig die genannten Nachteile der Hämofiltration entsprechend vermieden.

Durch die vorgeschlagene Vorrichtung ergibt sich auch der Vorteil, dass eine Verminderung der Behandlungseffizienz bzgl. der Mittelmoleküle durch die Zuführung des Fitrats aus dem zweiten Dialysator zu dem Blut des Patienten in Prädilution vermieden wird. Vielmehr wird das benutzte Dialysat teilweise regeneriert und als Dialysat wiederverwendet, wodurch im Übrigen frisches Dialysat eingespart werden kann, sodass die Behandlung kostengünstiger wird. Darüber hinaus ergibt sich durch die erfindungsgemäße Vorrichtung der Vorteil, dass das eingesetzte Dialysat den regulatorischen Anforderungen des Medizinproduktegesetztes mit den damit verbundenen Flexibilitätsvorteilen entsprechen muss, da es nicht direkt dem Blut zugegeben wird, sondern lediglich im Dialysatkreislauf zirkuliert. Es ist somit in zwei der Ausführungsformen kein Einsatz einer Substitutionslösung notwendig, die dem Arzneimittelgesetz unterliegt. Aufgrund der unterschiedlichen Zulassungswege bei einem Dialysat kann dieses schneller an medizinische Bedürfnisse angepasst werden, wie beispielsweise die Zugabe von Phosphat oder dergleichen. Außerdem stehen Substitutionsflüssigkeiten üblicherweise nur in Form von 5I-Beuteln zur Verfügung. Stattdessen ist vorliegend die Verwendung von Dialysat möglich, das online aus Konzentrat und Wasser hergestellt, aus einer Zentralversorgung bezogen oder als Batch mit ca. 30 - 100I am Behandlungsplatz zur Verfügung gestellt werden kann. Die Vorrichtung ist darüber hinaus auch für kleine Blutflüsse geeignet, wodurch weniger Alarme wegen Gefäßzugangsproblemen auftreten. Auch dies führt zu einer verbesserten Behandlung und geringerem Arbeitsaufwand. Da nur ein geringer konvektiver Stofftransport erfolgt, z. B. im Rahmen des notwendigen Wasserentzugs, bleibt der Filter bei dieser Vorrichtung typischerweise über einen längeren Zeitraum funktionstüchtig. Sowohl der Materialverbrauch als auch der Arbeitsaufwand werden hierdurch vermindert. Das auf Diffusion beruhende Verfahren, welches durch die Vorrichtung realisiert wird, vermindert den Albuminverlust für den Patienten. Das Verfahren erreicht mithin eine deutliche Verbesserung für den Patienten und die Wirtschaftlichkeit der Behandlung.

Bei einer bevorzugten Ausführungsform der Erfindung wird dem durch Filtration wiedergewonnenen Dialysat frisches Dialysat (folgend auch als Dialysatersatz bezeichnet) zugeführt, um den Retentatfluss auszugleichen. Die Zugabe von Dialysatersatz kann, in Flussrichtung des Dialysats gesehen, entweder vor oder nach dem zweiten Dialysator stattfinden. Besonders vorteilhaft ist die Zugabe des Dialysatersatzes vor dem zweiten Dialysator in dem extrakorporalen Dialysatkreislauf, weil der zweite Dialysator dann einerseits als Regenerator für das Dialysat des ersten Dialysators und andererseits als Dialysiatfilter zur Entfernung von Bakterien und Endotoxinen aus dem Dialysatersatz wirkt. Der zweite Dialysator weist dann also eine Doppelfunktion auf und wirkt zusätzlich als Sterilfilter für das frische Dialysat. Dadurch wird die Qualität des Dialysats gesteigert und ein zusätzlicher Dialysatfilter kann eingespart werden.

In einer alternativen Ausführungsform der Dialysevorrichtung ist eine Zuführung von Dialysatersatz in den Dialysatkreislauf in Flussrichtung nach dem zweiten Dialysator und vor den ersten Dialysator (3) vorgesehen ist. Hierdurch wird der Dialysatersatz effektiv genutzt, da er unmittelbar in den ersten Dialysator geleitet wird ohne dass seine Menge durch die Filtration im zweiten Dialysator reduziert wird.

Besonders vorteilhaft sind somit diese beiden Ausführungsformen, bei denen sowohl das teilweise regenerierte Dialysat also auch das frische Dialysat gemeinsam dem Dialysatraum des ersten Dialysators zur Wiederverwendung zugeführt werden. Von besonderem Vorteil ist hierbei jedoch die Zuführung des frischen Dialysats vor dem zweiten Dialysator in Flussrichtung des Dialysats gesehen, weil auf diese Weise ein zusätzlicher Filter zur Reinigung des frischen Dialysats eingespart werden kann. Die Reinigungsfunktion übernimmt der zweite Dialysator zusätzlich.

In einer weiteren alternativen Ausführungsform ist eine Zuführung einer Substitutionslösung in den Blutkreislauf vorgesehen. Diese Zuführung findet dabei vorteilhafter Weise in Flussrichtung gesehen vor dem ersten Dialysator statt. Dies bedeutet, dass eine Menge von Substitutionsflüssigkeit dem Blut zugeführt und im ersten Dialysator in den Dialysatkreislauf übertragen wird, welche weitgehend dem Retentatabfluss im zweiten Dialysator entspricht. Aufgrund der somit größeren übertragenen Menge im ersten Dialysator kann auch die Blutwäsche verbessert werden. Auch kann hierdurch die gewünschte Blutzusammensetzung direkter gesteuert werden und ggf. durch zusätzliche Medikamentengabe optimiert werden.

Vorteilhafter Weise weist der zweite Dialysator eine Filtermembran mit einem Cut-Off-Wert im Bereich von 1.000 - 15.000 Da auf und vorzugsweise von 5.000 - 10.000 Da. Auch kann es sich beim zweiten Dialysator um einen Mid-Flux-Dialysator mit einem Cut-Off-Wert von 15.000 - 20.000 Da handeln. Beim ersten Dialysator kann es sich um einen High-Flux-Dialysator mit einem Cut-Off-Wert von 20.000 - 40.000 Da handeln. Ein Dialysator mit 40 kDa als Cut-Off wird auch als High-Cut-Off-Dialysator bezeichnet. Die genannten Werte für den zweiten Dialysator bezeichnen eine untere Grenze des Molekulargewichts und die Werte des ersten Dialysators eine obere Grenze der Clearance. Der so definierte Molekularbereich wird verstärkt dem Blutfluss entzogen. Während kleinere Moleküle, also bspw. Elektrolyte, bei der Dialyse dem Blut weniger stark zu entziehen sind und Moleküle, die größer als die genannten Mittelmoleküle sind, - wie bspw. Albumin - dem Blut nicht entzogen werden sollen, so kann das Fenster bspw. auf interleukin oder Zytokine oder andere weitere zu entfernende Molekülgrößen eingestellt werden. Ein größerer Cut-Off-Wert des zweiten Dialysators von 5.000 Da oder 15.000 sorgt auch dafür, dass dessen Membranwiderstand nicht so schnell ansteigt und ausgetauscht werden muss.

Weiter kann die Dialysevorrichtung so eingerichtet sein, dass das Mengenverhältnis des Permetatstroms zu der Fitrationszufuhr größer 0,5 ist und vorzugsweise 0,8 +/- 0,1 beträgt. Dies bedeutet, dass der Retentatstrom 50% des zweiten Dialysators und vorzugsweise nur 20% +/- 10% beträgt. So wird ein großer Anteil des Dialysats im Kreislauf gehalten, was die Menge des Dialysatersatzes und somit die genannten Kosten reduziert.

Wie erläutert ist die Dialysevorrichtung bevorzugt geeignet, dem Blutstrom im ersten Dialysator überproportional viele Moleküle einer mittleren Masse zu entziehen. Diese befindet sich bspw. im Bereich der Mittelmoleküle von 1.000 - 50.000 Da und vorzugsweise 5.000 - 25.000 Da. Hierbei wird der Entzug der Moleküle auf den Entzug von Molekülen einer kleineren Masse also insbesondere kleiner 1000 Da bezogen.

Das Verhältnis des Cut-Offs des zweiten Dialysators zum ersten Dialysator liegt im Bereich von 1 zu 5 bis 1 zu 40. Auch kann dieses Verhältnis im Bereich von 1 zu 8 bis 1 zu 12 liegen. Die Beziehung der Cut-Off-Werte beider Dialysatoren zueinander ist deshalb relevant, da bei einem höheren Cut-Off des ersten Dialysators vorteilhafter Weise auch der Cut-Off des zweiten Dialysators höher auszulegen ist, um so ein vorzeitiges Verstopfen des zweiten Dialysators zu verhindern.

Auch kann der dem Blutstrom im ersten Dialysator entzogende Anteil der Moleküle einer Masse von 15.000 Da um mindestens 20% größer sein, als der entsprechende Anteil der Moleküle einer Masse von unter 1.000 Da. Da bspw. Zytokine eine Masse von 14 - 17 kDa aufweisen, wird durch diese Beziehung ausgedrückt, dass vermehrt diese Zytokine im Vergleich zu kleineren Molekülen dem Blutfluss entzogen werden.

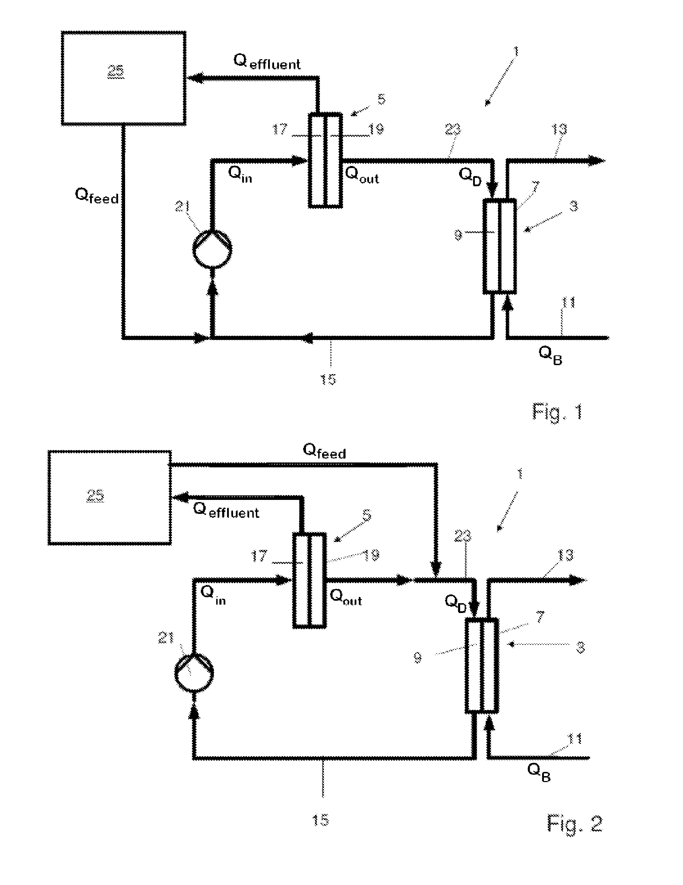

In einer weiteren vorteilhaften Ausführungsform weist der Dialysatkreislauf eine Heizung für das Dialysat auf. Üblicherweise wird bei bekannten Dialyseverfahren das zugeführte Dialysat (oder Dialysatersatz) auf ca. Bluttemperatur erwärmt. Üblicherweise wird Dialysatersatz mit einer Bikabonatkonzentration von 35 mmol/l verwendet, was leicht höher ist, als die im menschlichen Blut vorhandene Bikabonatkonzentration von 25 - 30 mmol/l. Eine hohe Bikabonatkonzentration ist aber nachteilig, da es mit vorhandenen Ionen (z.B. Mg⁺⁺ oder Ca⁺⁺) zu Ausfällungen kommen kann. So kann vorzugsweise das unerwärmte Dialysatersatz Q_{feed} dem Dialysatkreislauf zugeführt werden. Da dort die Bikabonatkonzentration geringer (vorzugsweise um 30 mmol/l oder leicht höher) ist, wird sich ein Mittelwert der Bikabonatkonzentration aus dem Dialysat des Kreislaufs und dem zugeführten Q_{feed} bilden. Wenn anschließend das Gemisch erwärmt wird, so ist die Ausfällungsneigung aufgrund der geringeren Konzentration geringer. Gemäß Fig. 1 kann die Heizung (nicht dargestellt) im Bereich der Pumpe liegen. So wird zuerst dem Dialysatkreislauf Q_{feed} zugeführt, über einen gewissen Leitungsabschnitt vermischt und anschließend auf die benötigte Bluttemperatur erwärmt.

Die Erfindung wird im Folgenden anhand der Zeichnung näher erläutert. Es zeigen:
- Fig. 1 bis Fig. 3: jeweils eine schematische Darstellung einer Ausführungsform der Dialysevorrichtung und
- Fig. 4: einen Clearance-Diagram am Beispiel von Dextranen.

Figur 1 zeigt eine Dialysevorrichtung 1 gemäß einer ersten Ausführungsform der Erfindung. Sie weist einen ersten Dialysator 3 und einen zweiten Dialysator 5 auf. Der erste Dialysator 3 umfasst einen Blutraum 7 und einen Dialysatraum 9, wobei beide Räume durch eine Membran voneinander getrennt sind. Dialysatoren der hier angesprochenen Art sind allgemein bekannt. Sie weisen ein Gehäuse auf, in dem beispielsweise mehrere zu einem Hohlfaserbündel zusammengefasste zylinderförmige Hohlfasermembranen vorgesehen sind. Durch das Innere der Hohlfasermembranen fließt Blut, während im Raum zwischen den Hohlfasermembranen und dem Filtergehäuse ein Dialysat vorzugsweise im Gegenstrom zum Blut fließt. Selbstverständlich können auch anders ausgebildete Dialysatoren vorgesehen sein. Dementsprechend wird vorliegend unter Blutraum 7 die Summe der Bereiche verstanden, in denen Blut im Dialysator 3 fließt und unter Dialysatraum 9 die Summe der Bereiche, in denen ein Dialysat fließt.

Die Wand der Membran des Dialysators 3 ist semipermeabel ausgebildet, sodass ein Stoffaustausch zwischen dem Blut und dem Dialysat erfolgen kann. Bei der Hämodialyse erfolgt dieser durch Diffusion, solange bis sich ein Gleichgewicht in dem Blutraum 7 und dem Dialysatraum 9 einstellt. Je nach der Porengröße der Membranen werden größere oder kleinere Moleküle in den Dialysatraum 9 durchgelassen.
Im Betrieb der Dialysevorrichtung 1 ist ein Patient an diese angeschlossen. Über eine Blutzuführung 11 fließt das Blut des Patienten in den Blutraum 7 des ersten Dialysators 3 und von dort über die Rückführung 13 zurück zum Patienten.

In dem Dialysatraum 9 des ersten Dialysators 3 fließt das Dialysat vorzugsweise im Gegenstrom zu dem Blutfluss, wie in Figur 1 durch die Pfeile angedeutet ist. Während der Dialyse findet der oben beschriebene Stoffaustausch zwischen dem Blut und dem Dialysat statt, wobei letzteres anschließend den Dialysator 3 über die Leitung 15 verlässt. Das Dialysat enthält dabei die Substanzen, die während der Dialyse aus dem Blutkreislauf Q_{B} entfernt werden.

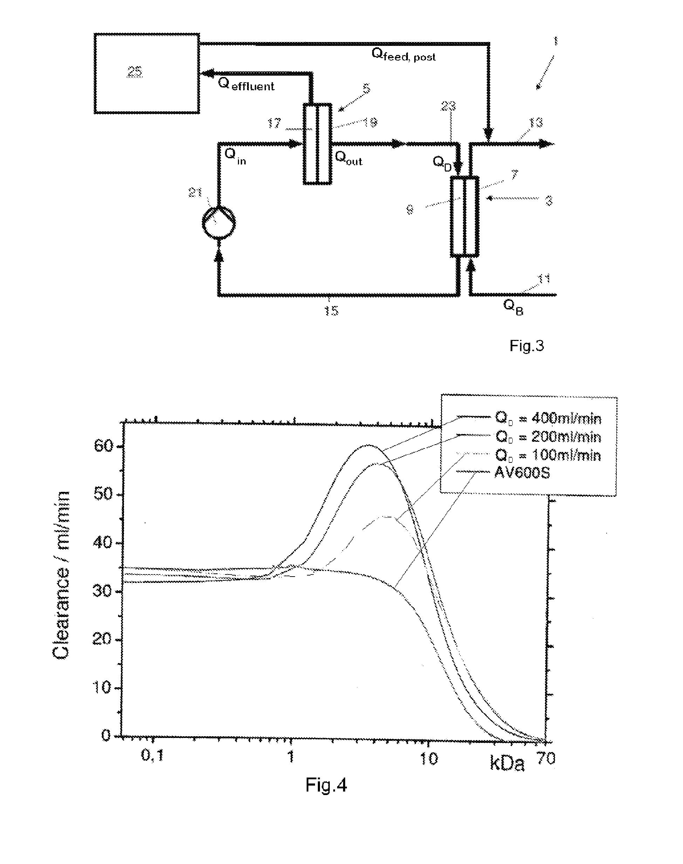

Wie eingangs erläutert wurde, ist es insbesondere bei akutem Nierenversagen wichtig, mittelgroße Moleküle (im Folgenden auch als Mittelmoleküle bezeichnet) aus dem Blutkreislauf des Patienten zu entfernen. Im vorliegenden Ausführungsbeispiel der Fig. 4 werden verstärkt Moleküle einer Masse von 1.000 bis 10.000 Da aus dem Blutstrom entfernt. Im erfinderischen Gedanken können auch Moleküle mit einer Masse von 1 kDa bis 50 kDa oder bis 70 kDa als mittelgroße Moleküle verstanden werden.

Hierzu ist der Dialysator 3 derart ausgebildet, dass er verstärkt Mittelmoleküle aus dem Blut entfernt, diese also in das Dialysat diffundieren können. Dabei ist der Dialysator 3 derart ausgebildet, dass seine Membran von der Porengröße auf den gewünschten Dalton-Cut-Off angepasst ist. Dalton dabei eine Masseeinheit und entspricht 1,6601 * 10⁻²⁷ kg.

Die Membran des ersten Dialysators 3 sorgt also dafür, dass Moleküle, die den entsprechenden Massegrenzwert überschreiten, nicht in den Dialysatkreislauf Q_{D} gelangen können. Wie später im Detail noch erläutert wird, bewirkt der zweite Dialysator 5, dass der ihn verlassene Permetatstrom Qₒᵤₜ , der als Dialysatfluss Q_{D} wieder in den Dialysatraum 9 geleitet wird, praktisch keine Moleküle mittlerer Masse, also bspw. mit einer Masse über 6.000 Da, aufweist. Er weist vielmehr eine gewisse Konzentration kleinerer Moleküle auf. Während in beiden Kammern des ersten Dialysators 3 ein geringer Konzentrationsgradient kleiner Moleküle (bis 1.000 Da) herrscht, kommt es bei diesem Gewichtsbereich nur zu einem gewissen Molekülübertritt in der Membran. Da aber die Konzentration mittelgroßer Moleküle im Dialysatfluss Q_{D} deutlich kleiner als im Blut ist, kommt es somit zu einem Konzentrationsausgleich dieser Moleküle mittlerer Masse. Bei größeren Molekülmassen, deren Masse oberhalb des Cut-offs der Membran des ersten Dialysators 3 liegt, wird hingegen der Konzentrationsausgleich durch die Permeabilität der Membran verhindert. So wird bewirkt, dass die Dialysevorrichtung dem Blut vornehmlich Moleküle mittlerer Masse entzieht.

Der Dialysators 3 kann beispielsweise als High-Flux-Dialysator, auch High-Flux-Filter genannt, ausgebildet sein. Auch kann vorliegend ein High-Cut-Off-Dialysator zum Einsatz kommen, der ebenfalls aus dem Stand der Technik bekannt ist und der eine noch größere Porengröße aufweist als ein High-Flux-Dialysator. Als Richtgröße kann angegeben werden, dass High-Flux einen Cut-Off von approx. 30 kDa +/- 10 kDa und High-Cut-Off-Filter (auch: Enhanced-Middle-Molecule-Clearance-Filter) einen Cut-Off von 40 kDa oder auch mehr aufweisen.

Über die Leitung 15 gelangt gemäß Fig. 1 das "benutzte" Dialysat aus dem ersten Dialysator 3 als Filtrationszufuhr Qᵢₙ in einen Eingangsraum 17 des zweiten Dialysators 5. Der Permetatstrom Qₒᵤₜ des zweiten Dialysators 5, also der Anteil der Filtrationszufuhr Qᵢₙ, welcher die Membran durchdringt, wird als Q_{D} dem ersten Dialysator 3 zugeführt und auf diese Weise wird der Dialysatkreislauf geschlossen. Anders als beim ersten Dialysator, welcher gemäß dem Prinzip der Hämodialyse mit zwei vorzugsweise entgegengesetzte Volumenströmen arbeitet, lässt sich der zweite Dialysator 5 auch als Filter bezeichnen, der den Strom der Filtrationszufuhr Qᵢₙ in zwei Ströme, also den Permetatstrom Qₒᵤₜ und den Retentatabfluss Q_{effulent} aufgespaltet. Wenn bspw. ein Filter mit einem Cut-Off von 1.000 Da verwendet wird, wird folglich der Permetatstrom Qₒᵤₜ im Wesentlichen keine Moleküle schwerer als 1.000 Da aufweisen, da diese im Filter zurückgehalten werden und den zweiten Dialysator 5 mit dem Retentatabfluss Q_{effluent} verlassen und einer Versorgungseinrichtung 25 zugeführt werden und dort gespeichert bzw. entsorgt werden.

Da auf diese Weise im zweiten Dialysator 5 dem Dialyatkreislauf Mittelmoleküle entzogen werden und kleinere Moleküle, also solche unterhalb seiner Cut-Off-Grenze, im Dialysatkreislauf belassen werden, bewirkt der zweite Dialysator 5 eine teilweise Regenerierung des Dialysats.

Das resultierende Filtrat, oder Permetatstrom Qₒᵤₜ, weist somit eine reduzierte Anzahl an Mittelmolekülen auf und gelangt von dem Filtratraum 19 über eine Leitung 23 zu dem Dialysatraum 9 des ersten Dialysators 3, wo es wiederum als Dialysierflüssigkeit bzw. als Dialysat verwendet wird. Dadurch, dass im zweiten Dialysator 5 die Anzahl der Mittelmoleküle reduziert wurde, wird nach der Rückführung des Dialysats bzw. Filtrats zu dem ersten Dialysator 3 ein Konzentrationsgradient zwischen dem Blut im Blutraum 7 und dem teilweise regenerierten Dialysat im Dialysatraum 9 bewirkt. Durch den Konzentrationsgradienten herrscht kein Gleichgewicht mehr zwischen der Konzentration der Mittelmoleküle in dem Dialysat und dem Blut, sodass wiederum vermehrt Mittelmoleküle durch die Membranen in das Dialysat diffundieren. Dieses Dialysat wird in dem Dialysatkreislauf wieder dem zweiten Dialysator 5 zugeführt und schließlich wieder teilweise als Dialysat verwendet und so weiter. Figur 1 macht deutlich, dass sich der Dialysatkreislauf von dem ersten Dialysator 3 über die Leitung 15 zu dem zweiten Dialysator 5 und weiter über die Leitung 23 zum ersten Dialysator 3 zurück erstreckt.

Der Volumenstrom des Retentatabflusses Q_{effluent}, welcher dem Dialsatkreislauf entzogen wurde, muss in Form von Dialysatersatz Q_{feed} zugeführt werden, wobei die Summe des Retentatabflusses Q_{effluent} und der resultierend im ersten Dialysator übertragenen Menge dem Dialysatersatz Q_{feed} weitgehend entsprechen muss, um die Menge innerhalb Dialysatkreislauf konstant zu halten. Bei beabsichtigten Volumenentzug aus dem Patientenblut wird Q_{effluent} größer als Q_{feed} eingestellt. Dabei entspricht die Differenz dem beabsichtigten Volumenentzug.

Im zweiten Dialysator 5 beträgt das Filtrationsverhältnis Qₒᵤₜ / Qᵢₙ, also das Verhältnis der Menge des Kaskadenausgangs (Qₒᵤₜ, also dem regenerierten Fluss) zu dem Kaskadeneingangsflusses (Qᵢₙ, dem Kaskadennfilter zugeführten Fluid) etwa 0,5 bis 1, vorzugsweise 0,5 - 0,9. Das Filtrat Qₒᵤₜ des zweiten Dialysators 5 wird somit vor (siehe Fig. 1) oder nach (siehe Fig. 2) dem ersten Dialysator 3 durch den Dialysatersatz Q_{feed} verdünnt, sodass der prozentuale Anteil an niedermolekularen Molekülen bei Einleitung in den Dialysatraum 9 des ersten Dialysator 3 sinkt. Somit entsteht durch die Zufuhr von Dialysatersatz ebenfalls ein Konzentrationsgefälle, welches die Diffusion von niedermolekularen Substanzen aus dem Blut in das Dialysat in dem ersten Dialysator 3 unterstützt. Die Clearance der niedermolekularen Substanzen wird somit im Wesentlichen auch durch die Menge Dialysatersatz bestimmt.

Typische Flüsse hierbei sind ein Blutfluss Q_{B} von 100 - 200 ml/min, ein Dialysatersatz Q_{Feed} = 10 - 100 ml/min, typischerweise 40 ml/min und ein Dialysatfluss Q_{D} im ersten Dialysator = 100 - 400 ml/min, typischerweise 200 ml/min. Besonders bevorzugt wird eine Filtrationsfraktion Qₒᵤₜ / Qᵢₙ = 160 / 200 = 0,8.

In der Ausführungsform der Fig. 1 speist die Versorgungsvorrichtung 25 den Dialysatersatz Q_{feed} in Flussrichtung des Dialysats gesehen vor dem zweiten Dialysator 5 in den Dialysekreislauf. Die Einspeisung der frischen Lösung vor dem zweiten Dialysator 5 hat den Vorteil, dass der zweite Dialysator 5 dann einerseits als Regenerator für das Dialysat des ersten Dialysators 3 und andererseits aber auch als Dialysatfilter zur Entfernung von möglicherweise in dem Dialysatersatz Q_{feed} vorhandenen Bakterien und Endotoxinen wirkt. Der zweite Dialysator 5 weist somit eine Doppelfunktion auf und wirkt zusätzlich als Sterilfilter für den Dialysierersatz Q_{feed}. Ferner kann ein üblicherweise für die Reinigung des frischen Dialysats, also dem Dialysatersatz, vorgesehene Filter eingespart werden.

Ein Beispiel soll die Wirkungsweise verdeutlichen: Bspw. soll ein Entzündungsmediator (welcher eine Masse von 14 - 17 kDa hat, wie bspw. Zytokine) aus dem Blut entfernt werden. Der Dialysatfluss Q_{D} weist eine deutlich niedrigere Konzentration von Mittelmolekülen, also im Größenbereich des Entzündungsmediators auf, was diese Moleküle im ersten Dialysator 3 verstärkt in das Dialysat diffundieren lässt. Wenn das Dialysat in den zweiten Dialysator 5 geleitet wird, sorgt dessen niedrigerer Cut-Off von bspw. 1 kDa dafür, dass der Entzündungsmediator in deutlich reduzierter Konzentration im den zweiten Dialysator verlassenden Permetatstrom Qₒᵤₜ, enthalten ist. So kann der Permetatstrom Qₒᵤₜ im Sinne eines Kreislaufs wieder als Dialysat für den ersten Dialysator 3 verwendet werden.
Albumin ist ein Molekül, welches nicht aus dem Blutfluss Q_{B} entfernt werden soll. Da Albumin ein Dalton-Gewicht von 68 kDa aufweist, kann es nur in klinisch unbedeutender Menge an dem ersten Dialysator entfernt werden, wenn dieser einen Cut-Off von bspw. 40 kDa aufweist.

Eine weitere Ausführungsform der Erfindung zeigt Figur 2. Gleiche Teile sind mit gleichen Bezugszeichen versehen, sodass insofern auf die Beschreibung zu Figur 1 verwiesen wird, um Wiederholungen zu vermeiden.

Wiederum ist eine Versorgungsvorrichtung 25 vorgesehen, die für eine Versorgung des Dialysatkreislaufs mit Dialysatersatz vorgesehen ist. Anders als bei der Ausführungsform nach Figur 1 wird in Figur 2 der Dialysatersatz Q_{feed} aber in die Leitung 23 zugeführt, also nach dem zweiten Dialysator 5 in Flussrichtung des Dialysats gesehen. Die Einspeisung ist dem zweiten Dialysator 5 bei dieser Ausführungsform also nachgeordnet. Dadurch wird die höchste Effizienz in Bezug auf den eingesetzten Dialysatersatz erzielt, jedoch entfällt hierdurch die Doppelfunktion des zweiten Dialysators 5 als zusätzlicher Sterilfilter.

Weiterhin kann gemäß Fig. 3 vorgesehen sein, dass statt der Zuführung von Dialysatersatz Q_{feed,post} in den Dialysatkreislauf eine frische Substitutionslösung vorzugsweise in einer Postdilution direkt in das Blut des Patienten geleitet wird. Dies geschieht in Flussrichtung gesehen nach dem ersten Dialysator 3. Allerdings entfällt hierdurch der Vorteil, dass ausschließlich Lösungen verwendet werden, die nicht unter das Arzneimittelgesetz fallen, welche somit weniger flexibel in Bezug auf die Änderung ihrer Zusammensetzung sind. Der Dialysatersatz kann alternativ (nicht dargestellt) auch in einer Prädilution als frische Subsitutionslösung dem Blut vor dem ersten Dialysator 3 zugeführt werden.

Die Vorrichtung kann im Übrigen sowohl mit systemischer Antikoagulation als auch mit regionaler Antikoagulation (Zitratantikoagulation) betrieben werden.

Insgesamt zeigt sich, dass durch die vorliegende Erfindung eine Kaskadendialysevorrichtung bzw. ein Kaskadendialyseverfahren realisiert wird, bei denen eine höhere Mittelmolekülclearance auch bei vergleichsweise geringem Blutfluss erzielt werden kann. Dem gegenüber ist insbesondere in der Postdilution bei herkömmlichen Verfahren die Filtrationsfraktion üblicherweise auf 20 - 30% beschränkt, da es ansonsten zu einer Aufkonzentrierung der Blutbestandteile im Dialysator kommt, was zu Clotting oder einer anderen Blutschädigung führen kann. Darüber hinaus sinkt im Falle der Prädilution die Effizient der eingesetzten Lösung bei den herkömmlichen Verfahren mit steigender Dosis durch den Verdünnungseffekt.

Versuche haben ferner gezeigt, dass eine deutliche Verbesserung der Entfernung der Mittelmoleküle mit der Kaskadenanalyse im Vergleich zu einer Hämofiltration mit dem gleichen Lösungseinsatz erreicht werden kann.

Fig. 4. zeigt ein Beispiel für die Wirkungsweise der Dialysiervorrichtung anhand von Dextranen. Bei Dextranen kann die Clearance über einen weiten Molekülgrößenbereich simultan einfach bestimmt werden. Bei diesen Messreihen wurde ein Blutfluss Q_{B} von 200 ml/min verwendet und der Zufluss frischen Dialysats (gemäß Fig. 1 vor dem zweiten Dialysator 5) auf 35 ml/min eingestellt. Der Dialysatstrom Q_{D} wird variiert und beträgt 100ml/min, 200ml/min oder 400ml/min. Der Cut-Off-Wert des zweiten Dialysators 5 entspricht 1 kDa und der des ersten Dialysators 3 beträgt 20 kDa. Im Bereich von 2 bis 10 kDa ist die Clearance, also der dem Blut entfernte Molekülstrom in dem gezeigten Mittelgrößenbereich deutlich erhöht.
Die Messwerte werden mit einer Hämofiltration mit dem AV600S verglichen, welche gemäß Fig. 4 keine erhöhten Clearance-Werte in dem bezeichneten Mittelbereich aufweist. Dieses Beispiel zeigt die prinzipielle Funktionsweise des Kaskadendialysators.

Merkmale unterschiedlicher Ausführungsformen sind frei miteinander kombinierbar.

### Bezugszeichenliste

- 1: Dialysevorrichtung
- 3: erster Dialysator
- 5: Zweiter Dialysator
- 7: Blutraum
- 9: Dialysatraum
- 11: Blutzuführung
- 13: Rückführung
- 15: Leitung
- 17: Eingangsraum
- 19: Filtratraum
- 21: Pumpe
- 23: Leitung
- 25: Versorgungsvorrichtung
- Q_{B}: Blutfluss
- Q_{D}: Dialysatfluss
- Q_{feed}: Dialysatersatz
- Q_{effluent}: Retentatabfluss
- Qₒᵤₜ: Permetatstrom
- Qᵢₙ: Filtrationszufuhr

## Patentansprüche

1. Dialysevorrichtung (1) mit einem Dialysatkreislauf, welcher einen ersten Dialysator (3) und einen zweiten Dialysator (5) beinhaltet, wobei der erste Dialysator (3) über eine Blutzuführung (11) und eine Rückführung (13) mit dem Blutkreislauf eines Patienten verbindbar ist,
bei der eine Ableitung für einen Retentatfluss (Q_{effluent}) aus dem Dialysatkreislauf an dem zweiten Dialysator (5) vorgesehen ist und ein Permetatstrom (Qₒᵤₜ) des zweiten Dialysators (5) im Dialysatkreislauf zu dem ersten Dialysator (3) geleitet wird
**dadurch gekennzeichnet, dass**
der zweite Dialysator (5) eine Filtermembran mit einem Cut-Off-Wert von mindestens 500 Da und der erste Dialysator (3) eine Filtermembran mit einem höheren Cut-Off-Wert als der zweite Dialysator (5) aufweist.

2. Dialysevorrichtung nach Anspruch 1, **dadurch gekennzeichnet, dass** eine Zuführung für Dialysatersatz (Q_{feed}) in den Dialysatkreislauf in Flussrichtung nach dem ersten Dialysator (3) und vor den zweiten Dialysator (5) vorgesehen ist.

3. Dialysevorrichtung nach Anspruch 1, **dadurch gekennzeichnet, dass** eine Zuführung von Dialysatersatz (Q_{feed}) in den Dialysatkreislauf in Flussrichtung nach dem zweiten Dialysator (5) und vor den ersten Dialysator (3) vorgesehen ist.

4. Dialysevorrichtung nach Anspruch 1, **dadurch gekennzeichnet, dass** eine Zuführung von einer Substitutionslösung in den Blutkreislauf (Q_{B}) vorgesehen ist.

5. Dialysevorrichtung nach einem der vorgehenden Ansprüche, **dadurch gekennzeichnet, dass** der zweite Dialysator (5) eine Filtermembran mit einem Cut-Off-Wert im Bereich von 1.000 - 15.000 Da, vorzugsweise 5.000 - 10.000 Da aufweist.

6. Dialysevorrichtung nach einem der vorgehenden Ansprüche 1 - 4, **dadurch gekennzeichnet, dass** es sich bei dem zweiten Dialysator (5) um einen Mid-Flux-Dialysator mit einem Cut-Off-Wert von 15.000 - 20.000 Da handelt.

7. Dialysevorrichtung nach einem der vorgehenden Ansprüche, **dadurch gekennzeichnet, dass** es sich bei dem ersten Dialysator (3) um einen High-Flux-Dialysator mit einem Cut-Off-Wert von 20.000 - 50.000 Da handelt, vorzugsweise 30.000 - 40.000 Da.

8. Dialysevorrichtung nach einem der vorgehenden Ansprüche, **dadurch gekennzeichnet, dass** das Mengenverhältnis des Permetatstroms (Qₒᵤₜ) zu der Fitrationszufuhr (Qᵢₙ) größer 0,5 vorzugsweise 0,8 +/- 0,1 ist.

9. Dialysevorrichtung nach einem der vorangegangenen Ansprüche **dadurch gekennzeichnet, dass** die Dialysevorrichtung geeignet ist, dem Blutstrom (Q_{B}) im ersten Dialysator (3) überproportional viele Moleküle einer Masse von 1.000 - 30.000 Da, vorzugsweise 5.000 - 25.000 Da zu entziehen.

10. Dialysevorrichtung nach einem der vorangegangenen Ansprüche **dadurch gekennzeichnet, dass** das Verhältnis des Cut-Offs des zweiten Dialysators (5) zum ersten Dialysator (3) im Bereich von 1 zu 1,5 bis 1 zu 40 beträgt.

11. Dialysevorrichtung nach Anspruch 10 **dadurch gekennzeichnet, dass** das Verhältnis des Cut-Offs des zweiten Dialysators (5) zum ersten Dialysator (3) im Bereich von 1 zu 2 bis 1 zu 10 beträgt.

12. Dialysevorrichtung nach einem der vorangegangenen Ansprüche **dadurch gekennzeichnet, dass** die Clearance im ersten Dialysator (3) für Moleküle einer Masse von 15.000 Da um mindestens 20% größer ist, als die Clearance der Moleküle einer Masse von unter 1.000 Da.

13. Dialysevorrichtung nach einem der vorangegangenen Ansprüche, **dadurch gekennzeichnet, dass** in dem Dialysatkreislauf eine Heizung für das Dialysat vorgesehen ist.

## Claims

1. A dialysis device (1) having a dialysate circuit comprising a first dialyzer (3) and a second dialyzer (5) the first dialyzer (3) being connectable to the blood circulation of a patient via a blood feed line (11) and a recirculation line (13),
wherein in the second dialyzer (5) an effluent to a retentate flow (Q_{effluent}) from the dialysate circuit is provided and a permetate stream (Qₒᵤₜ) of the second dialyzer (5) in the dialysate circuit is passed to the first dialyzer (3)
**characterized in that**
the second dialyzer (5) has a filter membrane having a cut-off value of at least 500 Da and the first dialyzer (3) has a filter membrane having a cut-off value higher than that of the second dialyzer (5).

2. The dialysis device according to claim 1 **characterized in that** introduction of dialysate substitute (Q_{feed}) into the dialysate circuit is provided in the flow direction downstream of the first dialyzer (3) and upstream of the second dialyzer (5).

3. The dialysis device according to claim 1 **characterized in that** introduction of dialysate substitute (Q_{feed}) into the dialysate circuit is provided in the flow direction downstream of the second dialyzer (5) and upstream of the first dialyzer (3).

4. The dialysis device according to claim 1 **characterized in that** introduction of substitute solution into the blood circulation (Q_{B}) is provided.

5. The dialysis device according to one of the preceding claims **characterized in that** the second dialyzer (5) has a filter membrane having a cut-off value ranging from 1.000 to 15.000 Da, preferably 5.000 to 10.000 Da.

6. The dialysis device according to one of the preceding claims 1 - 4 **characterized in that** the second dialyzer (5) is a mid-flux dialyzer having a cut-off value of 15.000 - 20.000 Da.

7. The dialysis device according to one of the preceding claims **characterized in that** the first dialyzer (3) is a high-flux dialyzer having a cut-off value of 20.000 - 50.000 Da, preferably 30.000 - 40.000 Da.

8. The dialysis device according to one of the preceding claims **characterized in that** the proportion of the permetate stream (Qout) to the filtration feed (Qᵢₙ) is greater than 0.5 preferably 0.8 +/- 0.1.

9. The dialysis device according to one of the preceding claims **characterized in that** the dialysis device is suitable for the removal of an overproportionally high level of molecules having molecular masses of 1.000 - 30.000 Da, preferably 5.000 - 25.000 Da from the blood stream (Q_{B}) in the first dialyzer (3).

10. The dialysis device according to one of the preceding claims **characterized in that** the cut-off ratio of the first second dialyzer (5) to the first dialyzer (3) is in the range of 1 to 1.5 to 1 to 40.

11. The dialysis device according to claim 10 **characterized in that** the cut-off ratio of the second dialyzer (5) to the first dialyzer (3) is in the range of 1 to 2 to 1 to 10.

12. The dialysis device according to one of the preceding claims **characterized in that** the clearance in the first dialyzer (3) for molecules of molecular masses of 15.000 Da is at least 20% higher than the clearance of the molecules of molecular masses below 1.000 Da.

13. The dialysis device according to one of the preceding claims **characterized in that** a heating device for the dialysate is provided in the dialysate circuit.

## Revendications

1. Dispositif de dialyse (1) comprenant un circuit de dialysat qui contient un premier dialyseur (3) et un deuxième dialyseur (5), le premier dialyseur (3) pouvant être connecté par le biais d'une alimentation sanguine (11) et d'une ligne de retour (13) au circuit sanguin d'un patient,
une conduite de sortie pour un flux de rétentat (Qeffluent) hors du circuit de dialysat étant prévue au niveau du deuxième dialyseur (5) et un écoulement de perméat (Qout) du deuxième dialyseur (5) étant guidé dans le circuit de dialysat jusqu'au premier dialyseur (3),
**caractérisé en ce que**
le deuxième dialyseur (5) présente une membrane filtrante avec une valeur de coupure d'au moins 500 Da et le premier dialyseur (3) présente une membrane filtrante avec une valeur de coupure plus élevée que celle du deuxième dialyseur (5).

2. Dispositif de dialyse selon la revendication 1, **caractérisé en ce qu'**une conduite d'alimentation pour un produit de remplacement de dialysat (Qfeed) est prévue dans le circuit de dialysat dans la direction du flux après le premier dialyseur (3) et avant le deuxième dialyseur (5).

3. Dispositif de dialyse selon la revendication 1, **caractérisé en ce qu'**une conduite d'alimentation d'un produit de remplacement de dialysat (Qfeed) est prévue dans le circuit de dialysat dans la direction du flux après le deuxième dialyseur (5) et avant le premier dialyseur (3).

4. Dispositif de dialyse selon la revendication 1, **caractérisé en ce qu'**une conduite d'alimentation d'une solution de substitution est prévue dans le circuit sanguin (QB).

5. Dispositif de dialyse selon l'une quelconque des revendications précédentes, **caractérisé en ce que** le deuxième dialyseur (5) présente une membrane filtrante avec une valeur de coupure dans la plage de 1000 à 15 000 Da, de préférence de 5000 à 10 000 Da.

6. Dispositif de dialyse selon l'une quelconque des revendications précédentes 1 à 4, **caractérisé en ce que** le deuxième dialyseur (5) est un dialyseur à flux moyen avec une valeur de coupure de 15 000 à 20 000 Da.

7. Dispositif de dialyse selon l'une quelconque des revendications précédentes, **caractérisé en ce que** le premier dialyseur (3) est un dialyseur à flux élevé avec une valeur de coupure de 20 000 à 50 000 Da, de préférence de 30 000 à 40 000 Da.

8. Dispositif de dialyse selon l'une quelconque des revendications précédentes, **caractérisé en ce que** le rapport quantitatif de l'écoulement de perméat (Qout) à l'alimentation de produit de filtration (Qin) est supérieur à 0,5, de préférence à 0,8 ± 0,1.

9. Dispositif de dialyse selon l'une quelconque des revendications précédentes, **caractérisé en ce que** le dispositif de dialyse est approprié pour prélever dans l'écoulement sanguin (QB) dans le premier dialyseur (3), de manière surproportionnelle, de nombreuses molécules ayant une masse de 1000 à 30 000 Da, de préférence de 5000 à 25 000 Da.

10. Dispositif de dialyse selon l'une quelconque des revendications précédentes, **caractérisé en ce que** le rapport de la coupure du deuxième dialyseur (5) au premier dialyseur (3) est compris dans la plage de 1 à 1,5 jusqu'à 1 à 40.

11. Dispositif de dialyse selon la revendication 10, **caractérisé en ce que** le rapport de la coupure du deuxième dialyseur (5) au premier dialyseur (3) est compris dans la plage de 1 à 2 jusqu'à 1 à 10.

12. Dispositif de dialyse selon l'une quelconque des revendications précédentes, **caractérisé en ce que** la clairance dans le premier dialyseur (3) pour des molécules d'une masse de 15 000 Da est supérieure d'au moins 20 % à la clairance pour des molécules d'une masse inférieure à 1000 Da.

13. Dispositif de dialyse selon l'une quelconque des revendications précédentes, **caractérisé en ce qu'**un chauffage est prévu pour le dialysat dans le circuit de dialysat.
